# EUROPÄISCHE PATENTSCHRIFT

(11) **EP 2 453 854 B1**
(45) Veröffentlichungstag und Bekanntmachung des Hinweises auf die Patenterteilung: **26.03.2014**
(21) Anmeldenummer: 10805395.0
(22) Anmeldetag: 23.11.2010
(51) Int. Cl.: A61F 9/013

(54) **VORRICHTUNG ZUM ANBRINGEN EINER MARKIERUNG AM MENSCHLICHEN AUGE**
DEVICE FOR APPLYING A MARKING TO THE HUMAN EYE
DISPOSITIF D'APPLICATION D'UN MARQUAGE SUR L'OEIL HUMAIN

(30) Priorität: 08.09.2010 DE 102010044764; 13.07.2010 DE 102010027051; 27.01.2010 DE 102010006009
(43) Veröffentlichungstag der Anmeldung: 23.05.2012
(73) Patentinhaber: Geuder AG, 69126 Heidelberg (DE)
(72) Erfinder: FATH, Hartmut, 69168 Wiesloch (DE); NEUHANN, Tobias, 80796 München (DE)
(74) Vertreter: Ullrich & Naumann
(86) Internationale Anmeldenummer: PCT/DE2010/001357
(87) Internationale Veröffentlichungsnummer: WO 2011/091777

(56) Entgegenhaltungen:
- DE-U1-202008 004 593
- US-A- 4 739 761
- US-A- 5 226 905
- US-A1- 2004 167 540
- US-A1- 2009 254 108
- US-B1- 6 217 596

## Beschreibung

Die Erfindung betrifft eine Vorrichtung zum Anbringen einer Markierung am menschlichen Auge, insbesondere auf der Hornhaut.

Es besteht die grundsätzliche Notwendigkeit zur Markierung der Hornhaut des menschlichen Auges, nämlich in Vorbereitung ophthalmologischer Eingriffe bei einer Korrektur des Astigmatismus, vorzugsweise zur passgenauen Fixierung einer Linse an der Iris.

Gattungsbildende Vorrichtungen sind bereits aus der Praxis bekannt, meist unter der Bezeichnung "Markierungsinstrument".

Aus der DE 20 2008 004 593 U1 ist ein gattungsbildendes Markierungsinstrument bekannt, wobei dieses Instrument zum Erzeugen von Markierungen für augenchirurgische Eingriffe dient. Das bekannte Instrument umfasst einen Markierungskopf, der schwenkbar mit einem Instrumentengriff verbunden ist. Ein am Instrumentenkopf vorgesehenes Markierungselement ist in seiner Winkellage relativ zu einem Lotgewicht verstellbar angeordnet, so dass Markierungen mit einer Winkeleinstellung relativ zur Lotrechten erzeugbar sind.

Die US 2009/0 254 108 A1 bildet jeweils den Oberbegriff der Ansprüche 1 und 2.

Zum Ankoppeln des Markierungskopfes an eine beliebige Halteeinrichtung umfasst der Markierungskopf besondere Kopplungsmittel, nämlich zum unmittelbaren oder mittelbaren Verbindung mit der Halteeinrichtung. Insbesondere sind die Kopplungsmittel zur Aufnahme und zum Einstecken eines Anschlussstücks ausgebildet, wobei das Anschlussstück der Halteeinrichtung oder einem zwischengeschalteten Adapter zugeordnet ist.

Im Rahmen der alternativen Ausgestaltung der erfindungsgemäßen Vorrichtung nach Anspruch 2 lässt sich eine direkte Ankopplung an eine Spaltlampe realisieren, wonach der Markierungskopf ohne Adapter an die Spaltlampe ankoppelbar ist. Entsprechend umfasst das Markierungselement einen integralen Einsteckbereich zur unmittelbaren Ankopplung an eine Halteeinrichtung bzw. an die Spaltlampe.

Bei der bekannte gattungsbildenden Vorrichtung handelt es sich ausschließlich um ein handgehaltenes chirurgisches Instrument. Der Markierungskopf ist fest mit dem Instrument verbunden. Aufgrund der festen Zuordnung des Markierungskopfes zu dem Instrumentengriff ist die Durchführung einer Markierung auf die Anwendung des konkreten Instruments begrenzt. Außerdem besteht stets - zwangsweise - ein Bezug zur Lotrechten, entsprechend der gewählten Einstellung. Außerdem ist die Einstellung der Winkellage des dort vorgesehenen Markierungselements aufwendig, erfordert nämlich ein separates Werkzeug, wobei zur Einstellung das gesamte Instrument auf einem Halteblock zu positionieren ist.

Der vorliegenden Erfindung liegt die Aufgabe zugrunde, eine Vorrichtung zum Anbringen einer Markierung am menschlichen Auge, insbesondere auf der Hornhaut des menschlichen Auges, derart auszugestalten und weiterzubilden, dass sie bei einfachster Konstruktion einfach in der Winkelposition einzustellen und sowohl in Verbindung mit handgehaltenen Instrumenten als auch in Verbindung mit herkömmlichen Geräten aus der Ophthalmologie anwendbar ist.

Die voranstehende Aufgabe ist durch die Merkmale der Patentansprüche 1 und 2 gelöst. Danach umfasst die erfindungsgemäße Vorrichtung einen Markierungskopf und eine den Markierungskopf tragende Halteeinrichtung. Der Markierungskopf ist mit einem Markierungselement und einem das Markierungselement in einer vorgebbaren bzw. veränderbaren Winkelposition haltenden Träger ausgestattet. Der Träger ist drehfest zur Halteeinrichtung oder einer Bezugslinie angeordnet, wobei der Markierungskopf von der Halteeinrichtung lösbar ist. Die Erfindung selbst wird durch die kennzeichnenden Teile charakterisiert.

Erfindungsgemäß ist erkannt worden, dass es von enormem Vorteil ist, wenn es sich bei dem Markierungskopf um ein unabhängig handhabbares Bauteil handelt, welches einer beliebigen Halteeinrichtung zuordenbar ist. Dabei ist wesentlich, dass der Markierungskopf das zur Markierung der Hornhaut dienende Markierungselement trägt, wobei die Winkelposition des Markierungskopfes und somit des Markierungselements gegenüber einer Halteeinrichtung oder einer Bezuglinie einstellbar ist. Mit anderen Worten ist der Markierungskopf gegenüber der Halteeinrichtung drehbar und von dieser lösbar bzw. austauschbar.

In vorteilhafter Weise umfasst das Markierungselement ein mit Markierungstinte benetzbares Markierungswerkzeug, vorzugsweise zur Markierung einer Geraden oder mindestens zweier Punkte oder Abschnitte einer Geraden. Mittels dort aufgebrachter Tinte lässt sich auf der Hornhaut eine Markierung anbringen, nämlich beispielsweise mittels wasserlöslicher Farbe bzw. Tinte.

Bei dem zur Markierung dienenden Markierungswerkzeug handelt es sich in besondere vorteilhafter Weise um zwei gezackte, vorzugsweise zumindest teilweise gebogene Markierungsflanken, die gegen die Hornhaut des Auges gedrückt werden. Zuvor ist die zur Markierung dienende Farbe auf die Oberfläche des Markierungs-werkzeugs aufzubringen. Dies kann über eine Art Stempelkissen, einen die Farbe enthaltenden Schwamm oder dgl. erfolgen.

Zur besonders einfachen Veränderung der Winkelposition des Markierungselements bzw. des dazu gehörenden Markierungswerkzeugs ist das Markierungselement an oder in einem inneren Drehring befestigt, wobei der Drehring in dem als äußerer Haltering ausgeführten Träger - per Hand oder mittels eines Werkzeugs - drehbar ist. Wenngleich die Verwendung eines Werkzeugs die Einstellung vereinfachen könnte, lässt sich das Markierungselement bzw. der innere Drehring mit dem Markierungswerkzeug einfach per Hand verstellen bzw. drehen, nämlich in dem äußeren Haltering.

Auch ist es denkbar, dass die Einstellung vor einer Ankopplung des Markierungskopfes an die Halteeinrichtung erfolgt, so dass die Drehung des inneren Drehrings per Finger bzw. Eingriff in den inneren Durchgang des Drehrings denkbar ist. Jedenfalls lässt sich die Winkelstellung bei abgekoppeltem Markierungskopf mühelos vornehmen.

In weiter vorteilhafter Weise umfasst der innere Drehring eine die Winkelposition symbolisierende Markierung. Der äußere Haltering kann mit einer Skalierung zum Anzeigen der Winkelposition, vorzugsweise im Bereich von 0 bis 180 Grad, ausgestattet sein. Ebenso ist eine umgekehrte Anordnung bzw. Zuordnung von Markierung und Skalierung denkbar. Wesentlich ist jedenfalls, dass die Kombination von innerem Drehring und äußerem Haltering für eine konstruktiv einfache, in idealer Weise per Hand betätigbare Einstellbarkeit sorgt, und zwar unabhängig von einer den Markierungskopf tragenden Halteeinrichtung.

In ganz besonders vorteilhafter Weise sind die Kopplungsmittel und/oder das Anschlussstück gegenseitig verrastbar, wobei die dazu dienenden Rastmittel vorzugsweise federkraftbeaufschlagt sind. Eine sichere Ankopplung ist dadurch möglich, und zwar ungeachtet der konkreten Halteeinrichtung. Wesentlich sind lediglich die aufeinander abgestimmten Kopplungsmittel bzw. Aufnahmen und Anschlussstücke.

In ganz besonders vorteilhafter Weise handelt es sich bei der Halteeinrichtung um ein herkömmliches ophthalmologisches Gerät, jedoch ohne dessen eigentlichen Funktionskopf. Stattdessen wird der Markierungskopf angekoppelt. Genauer gesagt kann es sich bei der Halteeinrichtung um eine sogenannte Spaltlampe bzw. um einen Tonometer handeln, der in der ophthalmologischen Praxis ohnehin vorhanden ist. Zur Ankopplung des Markierungskopfes sind besondere Kopplungsmittel erforderlich, um eine sichere Wirkverbindung zwischen dem eigentlichen Tonometer bzw. der Spaltlampe und dem Markierungskopf zu schaffen. Die Kopplungsmittel bzw. das Anschlussstück sind/ist geräteseitig dem ophthalmologischen Gerät anzupassen. Funktionsseitig sind Kopplungsmittel in Form eines besonderen Anschlussstücks vorzusehen, mit denen sich der Markierungskopf sicher verbinden lässt. Auch ist es denkbar, dass der Markierungskopf auf der Anschlussseite mit besonderen Kopplungsmitteln ausgestattet ist, die sich unmittelbar an die Spaltlampe bzw. an entsprechende Geräte-/Gehäuseteile der Spaltlampe ankoppeln lassen.

Alternativ ist es denkbar, dass der gleiche Markierungskopf mit seinen Kopplungsmitteln - über einen Adapter oder unmittelbar - mit einem handgehaltenen ophthalmologischen Instrument verbindbar ist, wobei es sich bei dem handgehaltenen ophthalmologischen Instrument vorzugsweise um ein Instrument mit einer sich zur horizontalen oder vertikalen ausrichtenden, schwerkraftbelastenden Pendelaufnahme handeln kann. Auch hier spielt die Ankoppelbarkeit und entsprechend die Austauschbarkeit des Markierungskopfes eine ganz besondere Rolle, um beispielsweise Markierungsköpfe mit unterschiedlich definierten Markierungswerkzeugen-wahlweise - einsetzen zu können.

Die leichte Entkoppelbarkeit des Markierungskopfes sorgt auch hier für eine bequeme Handhabung in der Verstellbarkeit der Winkellage des Markierungswerkzeugs.

Außerdem sei angemerkt, dass die Entfernbarkeit des Markierungskopfes dessen Handhabung bei der Reinigung begünstigt.

Es gibt nun verschiedene Möglichkeiten, die Lehre der vorliegenden Erfindung in vorteilhafter Weise auszugestalten und weiterzubilden. Dazu ist einerseits auf die den Patentansprüchen 1 und 2 nachgeordneten Patentansprüche und andererseits auf die nachfolgende Erläuterung zweier bevorzugter Ausführungsbeispiele der Erfindung anhand der Zeichnungen zu verweisen. In Verbindung mit der Erläuterung der bevorzugten Ausführungsbeispiele der Erfindung anhand der Zeichnung werden auch im Allgemeinen bevorzugte Ausgestaltungen und Weiterbildungen der Lehre erläutert. In der Zeichnung zeigen
- Fig.1: in einer schematischen Ansicht einen Markierungskopf zur Ankopplung an ein Bauteil einer Spaltlampe unter Zwischenschaltung eines Adapters,
- Fig. 2a, 2b, 2c: in schematischen Teilansichten den Gegenstand aus Fig. 1, wobei es sich bei dem ophthalmologischen Bauteil um ein Teil einer Spaltlampe handelt,
- Fig. 3: in schematischen Teilansichten ein weiteres Ausführungsbeispiel einer erfindungsgemäßen Vorrichtung, wobei dort der Markierer ohne Vorkehrung eines Adapters, mit einem integralen Einsteckbereich, direkt an eine Spaltlampe ankoppelbar ist,
- Fig. 4: in einer schematischen Ansicht den gleichen Markierungskopf wie aus Fig. 1, jedoch zur unmittelbaren Ankopplung an ein augenchirurgisches Handinstrument,
- Fig. 5a, 5b: in schematischen Ansichten den Gegenstand aus Fig. 4, wobei es sich bei der Halteeinrichtung um ein ophthalmologisches Handinstrument handelt und
- Fig. 6: in einer schematischen Ansicht ein weiteres Ausführungsbeispiel eines ophthalmologischen Handinstruments mit ankoppelbarem Markierungskopf, wobei der Markierungskopf unmittelbar an das Handinstrument ansteckbar ist.

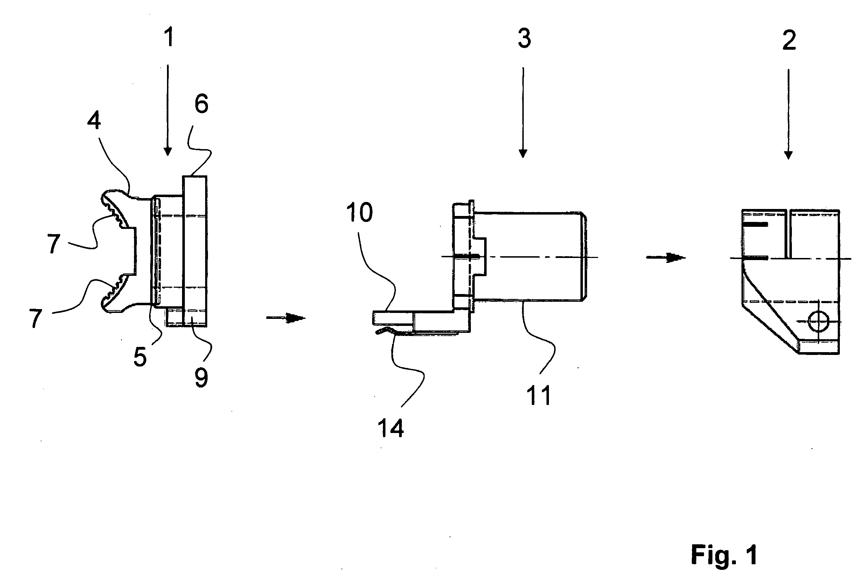

Fig. 1 zeigt in einer schematischen Ansicht die einzelnen Bestandteile einer erfindungsgemäßen Vorrichtung zum Anbringen einer Markierung am menschlichen Auge, nämlich auf der Hornhaut.

Die Vorrichtung umfasst einen Markierungskopf 1 und eine den Markierungskopf 1 tragende Halteeinrichtung 2, wobei es sich dabei um ein Bauteil einer herkömmlichen Spaltlampe bzw. eines Tonometers handelt.

Die Ankopplung des Markierungskopfes 1 an das Halteelement 2 erfolgt über Kopplungsmittel 3 in Form eines Adapters.

Der Markierungskopf 1 ist mit einem Markierungselement 4 ausgestattet, welches an einem inneren Drehring 5 befestigt ist. Der innere Drehring 5 ist gegenüber einem äußeren Haltering 6 drehbar, wobei die beiden Ringe - innerer Drehring 5 und äußerer Haltering 6 - gegeneinander drehbar sind.

Fig. 1 lässt des Weiteren erkennen, dass das Markierungselement 4 des Markierungskopfes 1 mit zwei Klauen 7 mit gezackten Markierungsflanken ausgestattet ist. Außerdem umfasst der Markierungskopf 1 eine Aufnahme 9 zum Einschieben bzw. Einstecken eines Anschlussstücks 10, welches integraler Bestandteil des Adapters 3 ist. Somit lässt sich eine sichere Wirkverbindung zwischen dem Markierungskopf 1 und dem Adapter 3 herstellen. Der Adapter 3 umfasst einen Einsteckbereich 11, der sich in das die Halteeinrichtung 2 symbolisierende Teil der Spaltlampe einstecken lässt. Somit ist es möglich, den Markierungskopf 1 auf einfache Weise an eine herkömmliche Spaltlampe anzuschließen und das gesamte Gerät zur Markierung der Hornhaut des menschlichen Auges zu nutzen, ähnlich der Anwendung einer herkömmlichen Spaltlampe bzw. eines Tonometers.

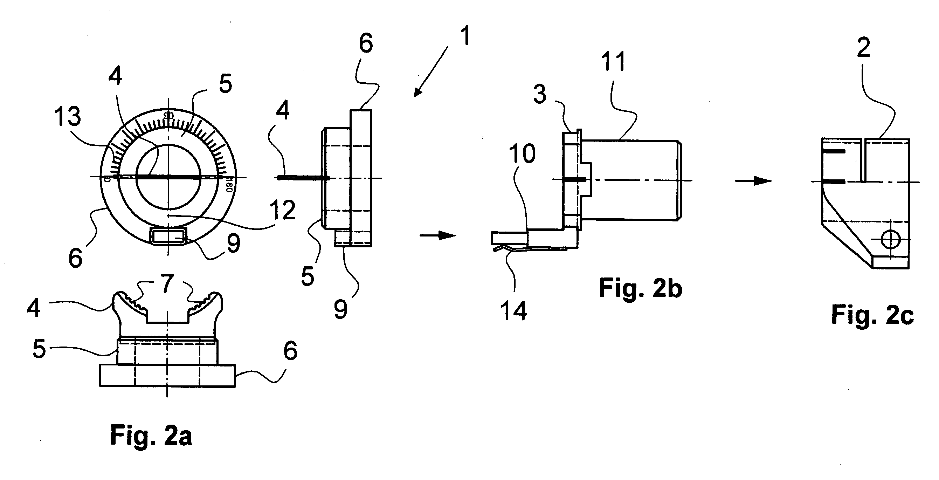

Die Fig. 2a, 2b und 2c zeigen den in die Halteeinrichtung 2 eingesteckten Adapter 3, wobei das Anschlussstück 10 zum Einstecken in die Aufnahme 9 des Markierungskopfes 1 dient. Die Aufnahme 9 kann als metallische Hülse bzw. als entsprechendes Hohlprofil ausgeführt sein.

Fig. 2a zeigt deutlich den Markierungskopf 1, bestehend aus dem inneren Drehring 5 mit Markierung 12 und dem äußeren Haltering 6 mit einer die Winkelstellung definierenden Skala 13. Die Skala 13 umfasst eine Einteilung von 0 bis 180 Grad, so dass beliebige Winkelstellungen des Markierungselements 4 bzw. der das Markierungswerkzeug definierenden Klauen 7 möglich sind.

Die Fig. 2a, 2b und 2c zeigen des Weiteren deutlich, dass das Anschlussstück 10 als in die Aufnahme 9 einsteckbares Profil ausgeführt ist, wobei das Anschlussstück 10 eine Federklammer 14 zur Verrastung bzw. Absicherung der Verbindung zwischen dem Adapter 3 und dem Markierungskopf 11 umfasst.

Die wesentlichen Teile des Markierungskopfes 1 können aus Kunststoff gefertigt sein. Das Markierungselement 4 bzw. die Klauen 7 sind in vorteilhafter Weise aus Metall gefertigt. Gleiches gilt für die Aufnahme 9, des Markierungskopfes 1. Auch das Anschlussstück 10 des Adapters 3 sollte aus Metall, vorzugsweise aus Aluminium oder Edelstahl, gefertigt sein. Bei dem Adapter 3 kann es sich ansonsten um ein im Wesentlichen aus Kunststoff gefertigtes Bauteil handeln.

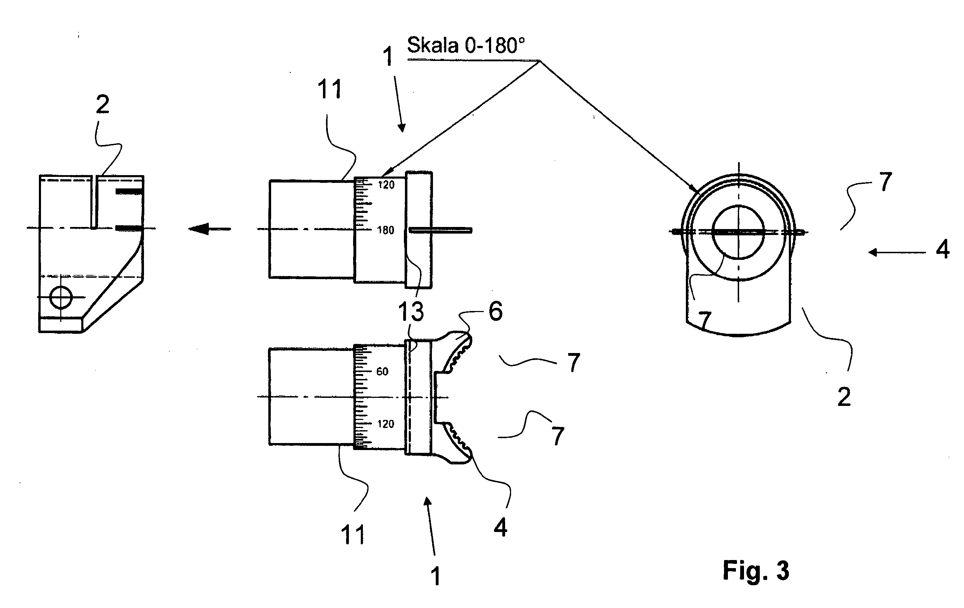

Fig. 3 zeigt ein weiteres Ausführungsbeispiel einer erfindungsgemäßen Vorrichtung, nämlich eine gegenüber der in den Fig. 1 und 2 gezeigten Vorrichtung modifizierte Ausführung zur unmittelbaren Ankopplung an eine Spaltlampe. Genauer gesagt lässt sich gemäß Fig. 3 der sogenannte Cornea-Markierer, d.h. der Markierungskopf 1, direkt an einer Spaltlampe anbringen, ohne Zwischenschaltung eines Adapters. Genauer gesagt umfasst der Markierungskopf 1 einen integralen Einsteckbereich 11 zum unmittelbaren Einstecken in die Halteeinrichtung 2. Alle weiteren Bestandteile der Vorrichtung sind entsprechend der Ausführungsform gemäß den Fig. 1 und 2 mit den gleichen Bezugszeichen versehen, so dass sich weitere Ausführungen unter Hinweis auf die Beschreibung zu den Fig. 1 und 2 erübrigen.

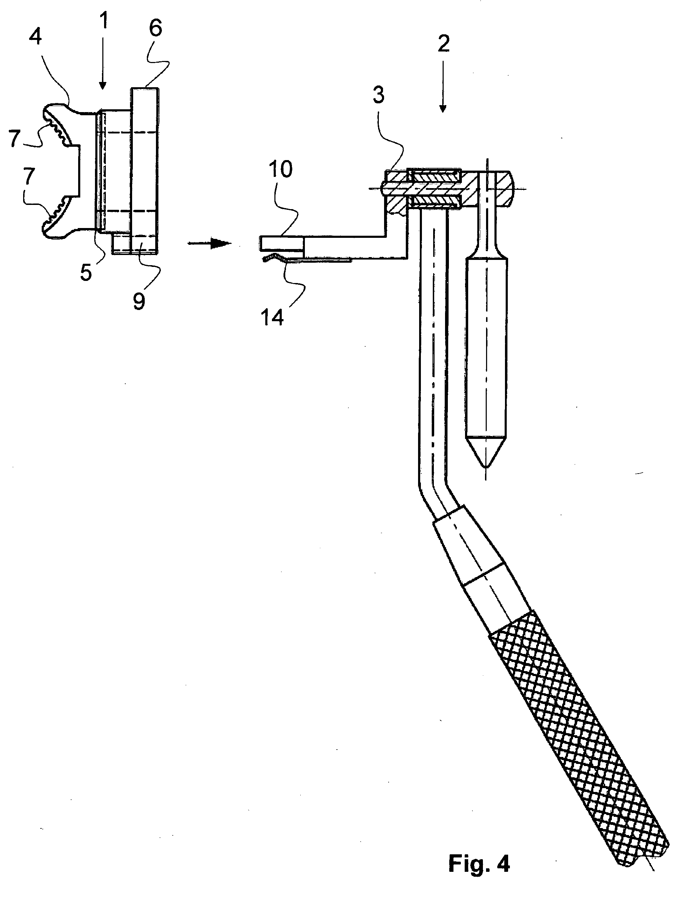

Fig. 4 zeigt ein drittes Ausführungsbeispiel einer erfindungsgemäßen Vorrichtung, wobei der Markierungskopf 1 identisch zu dem in den Fig. 1, 2a, 2b und 2c gezeigten Markierungskopf 1 ist. Über die Aufnahme 9 ist der Markierungskopf 1 unmittelbar mit dem Anschlussstück 10 eines Handinstruments verbindbar, wobei es sich um ein beliebiges Handinstrument mit einem entsprechend ausgebildeten Anschlussstück 10 handeln kann. In Verbindung mit dem Ausführungsbeispiel gemäß den Fig. 1, 2a, 2b und 2c lässt sich die vielseitige Verwendbarkeit des Markierungskopfes 1 darstellen, wobei eine einwandfreie Kopplung zwischen dem Markierungskopf 1 und einer beliebigen Halteeinrichtung 2 zu bewerkstelligen ist.

Bei dem in Fig. 4 gezeigten Ausführungsbeispiel ist die Halteeinrichtung 2 als handgehaltenes ophthalmologisches Instrument mit schwerkraftbelasteter Pendelaufnahme für den Markierungskopf 1 ausgebildet.

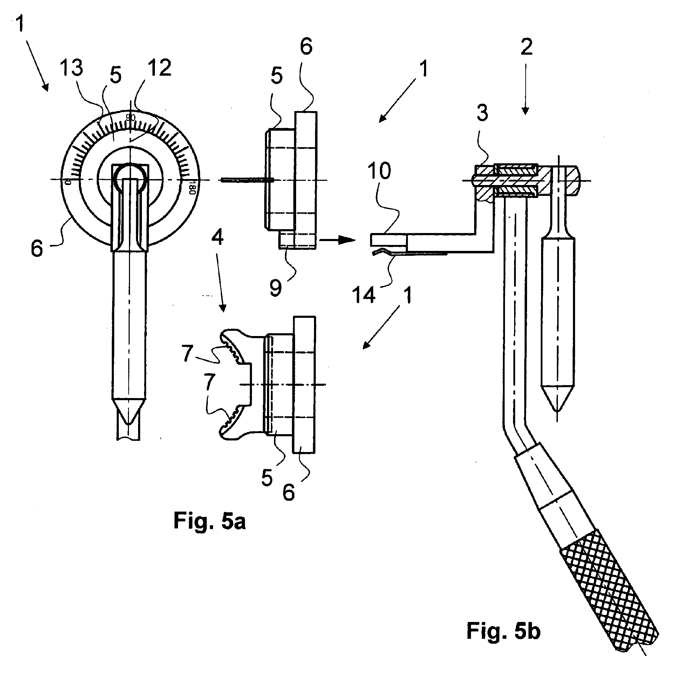

Die Fig. 5a und 5b zeigen das Ausführungsbeispiel aus den Fig. 4a, 4b in detaillierter Seitenansicht, wobei gemäß Fig. 5a der Markierungskopf 1 vom Handinstrument bzw. von der Halteeinrichtung 2 entkoppelt ist. Fig. 5b zeigt die Halteeinrichtung 2, die von einem sogenannten Pendelmarkierer getragen ist. Der Halteeinrichtung 2 bzw. dem Pendelmarkierer ist ein Anschlussstück 10 zugeordnet, welches eine zur Sicherung dienende Federklammern 14 umfasst. Das Anschlussstück 10 mit der Federklammer 14 dient zum Einschieben/Einstecken in eine dem Markierungskopf 1 zugeordnete Aufnahme 9.

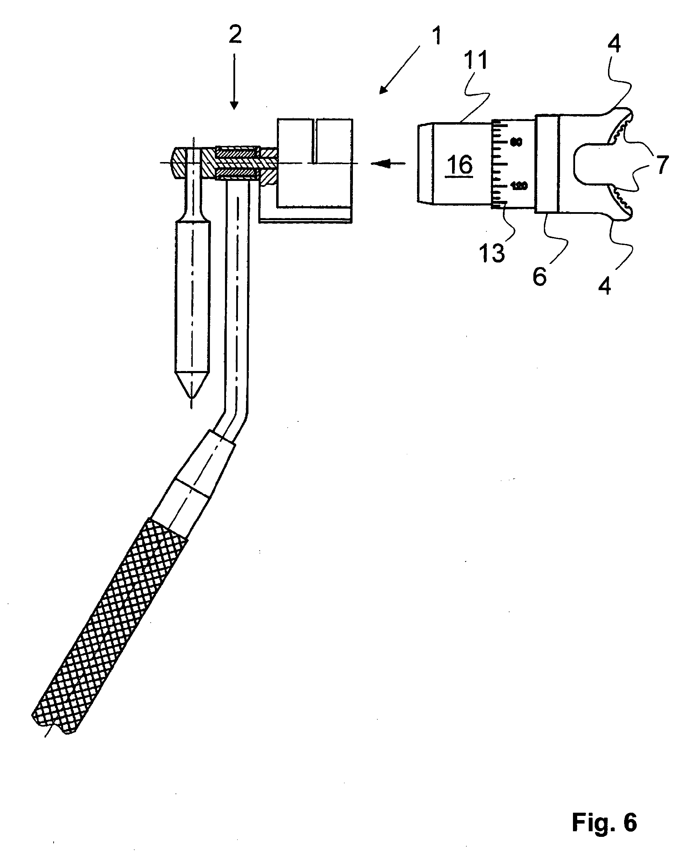

Fig. 6 zeigt ein weiteres Ausführungsbeispiel einer erfindungsgemäßen Vorrichtung, im Konkreten einen sogenannten Pendelmarkierer. Der grundsätzliche Aufbau entspricht dem Ausführungsbeispiel aus den Fig. 4 und 5. Im Gegensatz zu dem Ausführungsbeispiel aus den Fig. 4 und 5 ist das Ausführungsbeispiel gemäß Fig. 6 mit einem integralen Konnektor 15 ausgestattet, der im Sinne einer geschlitzten Hülse ausgebildet ist.

Der Markierungskopf 1 lässt sich mit einem Anschlussstück 16, das einen Einsteckbereich 11 umfasst, in den Konnektor 15 einstecken und dort festlegen bzw. arretieren.

Zu dem Konnektor 15 sei angemerkt, dass dessen Wandung im vorderen Bereich geschlossen und weiter hinten, d.h. auf der der Einsteckseite abgewandten Seite, geschlitzt ist. Die Wandung wirkt wie eine Feder, so dass sich das Anschlussstück 16 unter Nutzung der materialspezifischen Spannung in den Konnektor 15 einstecken und dort unter Materialspannung halten lässt.

Die sonstigen Bauteile des in Fig. 6 gezeigten Ausführungsbeispiels entsprechen ansonsten den Bauteilen des Ausführungsbeispiels aus den Fig. 4 und 5.

Schließlich sei angemerkt, dass es sich bei der Halteeinrichtung um beliebige Gerätschaften im Sinne von handgehaltenen Instrumenten oder Standgeräten handeln kann, die zur Positionierung des Markierungskopfes gegenüber dem menschlichen Auge geeignet sind.

Voranstehende Ausführungen dienen lediglich der beispielhaften Erörterung der beanspruchten Lehre, schränken diese jedoch nicht auf die beiden Ausführungsbeispiele ein.

### Bezugszeichenliste

- 1: Markierungskopf
- 2: Halteeinrichtung
- 3: Kopplungsmittel/Adapter
- 4: Markierungselement/Markierungswerkzeug
- 5: innerer Drehring
- 6: äußerer Haltering
- 7: Klaue
- 8: Markierungsflanke
- 9: Aufnahme
- 10: Anschlussstück
- 11: Einsteckbereich
- 12: Markierung
- 13: Skala
- 14: Federklammer
- 15: Konnektor
- 16: Anschlussstück

## Patentansprüche

1. Vorrichtung zum Anbringen einer Markierung am menschlichen Auge, insbesondere auf der Hornhaut, mit einem Markierungskopf (1) und einer den Markierungskopf (1) tragenden Halteeinrichtung (2), wobei der Markierungskopf (1) ein Markierungselement (4) und einen das Markierungselement (4) in einer vorgebbaren bzw. veränderbaren Winkelposition haltenden Träger umfasst, wobei der Träger drehfest zur Halteeinrichtung (2) oder einer Bezugslinie angeordnet ist und wobei der Markierungskopf (1) von der Halteeinrichtung (2) lösbar ist,
**dadurch gekennzeichnet, dass** der Markierungskopf (1) Kopplungsmittel (3) zum unmittelbaren oder mittelbaren Verbinden mit der Halteeinrichtung (2) zum Einstecken eines Anschlussstücks (10), umfasst, wobei das Anschlussstück der Halteeinrichtung (2) oder einem zwischengeschalteten Adapter zugeordnet ist.

2. Vorrichtung zum Anbringen einer Markierung am menschlichen Auge, insbesondere auf der Hornhaut, mit einem Markierungskopf (1) und einer den Markierungskopf (1) tragenden Halteeinrichtung (2), wobei der Markierungskopf (1) ein Markierungselement (4) und einen das Markierungselement (4) in einer vorgebbaren bzw. veränderbaren Winkelposition haltenden Träger umfasst, wobei der Träger drehfest zur Halteeinrichtung (2) oder einer Bezugslinie angeordnet ist und wobei der Markierungskopf (1) von der Halteeinrichtung (2) lösbar ist,
**dadurch gekennzeichnet, dass** der Markierungskopf (1) ein Anschlussstück (16) mit einem Einsteckbereich (11) zur unmittelbaren Ankopplung an die Halteeinrichtung (2), nämlich zum unmittelbaren Einstecken in einen im Sinne einer geschlitzten Hülse ausgebildeten Konnektor (15) der Halteeinrichtung (2), umfasst.

3. Vorrichtung nach Anspruch 1 oder 2, **dadurch gekennzeichnet, dass** das Markierungselement (1) ein mit Markierungstinte benetzbares Markierungswerkzeug (4), vorzugsweise zur Markierung einer Geraden oder mindestens zweier Punkte oder Abschnitte einer Geraden, ausgebildet ist.

4. Vorrichtung nach Anspruch 3, **dadurch gekennzeichnet, dass** das Markierungswerkzeug (4) zwei gezackte, vorzugsweise zumindest teilweise gebogene Markierungsflanken (8) umfasst.

5. Vorrichtung nach einem der Ansprüche 1 bis 4, **dadurch gekennzeichnet, dass** das Markierungselement (4) an oder in einem inneren Drehring (5) befestigt ist, der in dem als äußerer Haltering (6) ausgeführten Träger - per Hand oder mittels eines Werkzeugs - drehbar ist.

6. Vorrichtung nach Anspruch 5, **dadurch gekennzeichnet, dass** der innere Drehring (5) eine die Winkelposition symbolisierende Markierung und der äußere Haltering (6) eine Skalierung zum Anzeigen der Winkelposition, vorzugsweise im Bereich von 0 bis 180 Grad, umfasst.

7. Vorrichtung nach einem der Ansprüche 1-6 **dadurch gekennzeichnet, dass** die Kopplungsmittel (3) und/oder das Anschlussstück (10) vorzugsweise federkraftbeaufschlagte Rastmittel zum gegenseitigen Verrasten umfassen.

8. Vorrichtung nach einem der Ansprüche 1 bis 7, **dadurch gekennzeichnet, dass** als Halteeinrichtung (2) ein herkömmliches ophthalmologisches Gerät, ohne dessen eigentlichen Funktionskopf, beispielsweise eine Spaltlampe, dient.

9. Vorrichtung nach Anspruch 8, **dadurch gekennzeichnet, dass** der Markierungskopf (1) über ein Kopplungsmittel (3) bzw. ein Anschlussstück (10) umfassenden Adapter (3) mit dem Gerät verbunden ist.

10. Vorrichtung nach einem der Ansprüche 1 bis 7, **dadurch gekennzeichnet, dass** als Halteeinrichtung (2) ein handgehaltenes ophthalmologisches Instrument, vorzugsweise mit einer sich zur Horizontalen oder Vertikalen ausrichtenden, schwerkraftbelasteten Pendelaufnahme für den Markierungskopf (1), dient.

## Claims

1. Device for applying a marking to the human eye, in particular on the cornea, having a marking head (1) and a holding device (2) carrying the marking head (1), wherein the marking head (1) comprises a marking element (4) and a carrier that holds the marking element (4) in a specifiable, or changeable, angular position, wherein the carrier is arranged to be rotationally secure relative to the holding device (2) or a reference line, and wherein the marking head (1) is removable from the holding device (2),
**characterised in that** the marking head (1) comprises coupling means (3) for direct or indirect connection to the holding device (2) for insertion of a connecting piece (10), wherein the connecting piece is associated with the holding device (2) or with an intermediate adapter.

2. Device for applying a marking to the human eye, in particular on the cornea, having a marking head (1) and a holding device (2) carrying the marking head (1), wherein the marking head (1) comprises a marking element (4) and a carrier that holds the marking element (4) in a specifiable, or changeable, angular position, wherein the carrier is arranged to be rotationally secure relative to the holding device (2) or a reference line, and wherein the marking head (1) is removable from the holding device (2),
**characterised in that** the marking head (1) comprises a connecting piece (16) having an insertion region (11) for direct coupling to the holding device (2), namely for direct insertion into a connector (15), configured as a slotted sleeve, of the holding device (2).

3. Device according to claim 1 or 2, **characterised in that** the marking element (1) is in the form of a marking tool (4) which can be wetted with marking ink, preferably for marking a straight line or at least two points or sections of a straight line.

4. Device according to claim 3, **characterised in that** the marking tool (4) comprises two serrated, preferably at least partially curved, marking edges (8).

5. Device according to any one of claims 1 to 4, **characterised in that** the marking element (4) is fastened on or in an inner rotating ring (5) which is rotatable - by hand or by means of a tool - in the carrier in the form of an outer holding ring (6).

6. Device according to claim 5, **characterised in that** the inner rotating ring (5) comprises a marking symbolising the angular position and the outer holding ring (6) comprises a scale for indicating the angular position, preferably in the range from 0 to 180 degrees.

7. Device according to any one of claims 1 to 6, **characterised in that** the coupling means (3) and/or the connecting piece (10) preferably comprise spring-loaded locking means for mutual locking.

8. Device according to any one of claims 1 to 7, **characterised in that** there is used as the holding device (2) a conventional ophthalmological device without its actual functional head, for example a slit lamp.

9. Device according to claim 8, **characterised in that** the marking head (1) is connected to the device by way of an adapter (3) comprising coupling means (3) or a connecting piece (10).

10. Device according to any one of claims 1 to 7, **characterised in that** there is used as the holding device (2) a hand-held ophthalmological instrument, preferably having for the marking head (1) a gravity-loaded pendulum receiver which aligns itself to the horizontal or vertical.

## Revendications

1. Dispositif destiné à l'application d'un marquage sur un oeil humain, en particulier sur la cornée, comprenant une tête de marquage (1) et un dispositif de maintien (2) supportant la tête de marquage (1), étant entendu que la tête de marquage (1) comprend un élément de marquage (4) et un support maintenant l'élément de marquage (4) dans une position d'angle pouvant être prédéfinie ou modifiée, étant entendu que le support est agencé de façon non rotative par rapport au dispositif de maintien (2) ou à une ligne de référence et étant entendu que la tête de marquage (1) peut être détachée du dispositif de maintien (2),
**caractérisé en ce que** la tête de marquage (1) comprend des moyens de couplage (3) permettant de réaliser une jonction directe ou indirecte avec le dispositif de maintien (2) aux fins de l'insertion d'une pièce de raccordement (10), étant entendu que la pièce de raccordement est adjointe au dispositif de maintien (2) ou à un adaptateur monté en position intermédiaire.

2. Dispositif destiné à l'application d'un marquage sur un oeil humain, en particulier sur la cornée, comprenant une tête de marquage (1) et un dispositif de maintien (2) supportant la tête de marquage (1), étant entendu que la tête de marquage (1) comprend un élément de marquage (4) et un support maintenant l'élément de marquage (4) dans une position d'angle pouvant être prédéfinie ou modifiée, étant entendu que le support est agencé de façon non rotative par rapport au dispositif de maintien (2) ou à une ligne de référence et étant entendu que la tête de marquage (1) peut être détachée du dispositif de maintien (2),
**caractérisé en ce que** la tête de marquage (1) comprend une pièce de raccordement (16) ayant une zone d'insertion (11) aux fins du couplage direct avec le dispositif de maintien (2), notamment pour l'insertion directe dans un connecteur (15) du dispositif de maintien (2) réalisé à la manière d'une douille fendue.

3. Dispositif selon la revendication 1 ou 2, **caractérisé en ce que** l'élément de marquage (1) est réalisé comme un outil de marquage pouvant être chargé d'encre de marquage (4), de préférence aux fins du marquage d'une droite ou d'au moins deux points ou de segments d'une droite.

4. Dispositif selon la revendication 3, **caractérisé en ce que** l'outil de marquage (4) comprend deux flancs de marquage dentelés (8), de préférence au moins partiellement courbés.

5. Dispositif selon l'une des revendications 1 à 4, **caractérisé en ce que** l'élément de marquage (4) est fixé sur ou dans une bague rotative intérieure (5), qu'on peut faire tourner à la main ou au moyen d'un outil dans le support réalisé comme bague de maintien extérieure (6).

6. Dispositif selon la revendication 5, **caractérisé en ce que** la bague rotative intérieure (5) comprend un marquage symbolisant la position d'angle et la bague de maintien extérieure (6) une graduation destinée à indiquer la position d'angle, de préférence dans l'intervalle de 0 à 180 degrés.

7. Dispositif selon l'une des revendications 1 à 6, **caractérisé en ce que** les moyens de couplage (3) et/ou la pièce de raccordement (10) comprennent de préférence des moyens d'enclenchement soumis à un mécanisme à ressort aux fins d'un enclenchement réciproque.

8. Dispositif selon l'une des revendications 1 à 7, **caractérisé en ce qu'**un appareil ophtalmologique traditionnel sans sa tête fonctionnelle spécifique, par exemple une lampe à fente, fait fonction de dispositif de maintien (2).

9. Dispositif selon la revendication 8, **caractérisé en ce que** la tête de marquage (1) est relié avec l'appareil par le biais d'un adaptateur (3) comprenant un moyen de couplage (3) ou une pièce de raccordement (10).

10. Dispositif selon l'une des revendications 1 à 7, **caractérisé en ce qu'**un instrument ophtalmologique à main, ayant de préférence un support flottant soumis à la force de gravité s'orientant par rapport à l'horizontale ou à la verticale, fait fonction de dispositif de maintien (2) pour la tête de marquage (1).
